(19) Europäisches Patentamt
European Patent Office
Office européen des brevets

(11) Veröffentlichungsnummer: **0 019 762**
**B1**

(12) # EUROPÄISCHE PATENTSCHRIFT

(45) Veröffentlichungstag der Patentschrift: **24.03.82**

(51) Int. Cl.³: **C 07 D 249/08**, A 01 N 43/64

(21) Anmeldenummer: **80102485.2**

(22) Anmeldetag: **07.05.80**

(54) **Triazolylderivate, Verfahren zu ihrer Herstellung sowie diese enthaltende Mittel zur Beeinflussung des Pflanzenwachstums.**

(30) Priorität: **25.05.79 DE 2921168**

(43) Veröffentlichungstag der Anmeldung: **10.12.80 Patentblatt 80/25**

(45) Bekanntmachung des Hinweises auf die Patenterteilung: **24.03.82 Patentblatt 82/12**

(84) Benannte Vertragsstaaten: **AT BE CH DE FR GB IT LI LU NL SE**

(56) Entgegenhaltungen:
**EP-A-0 000 940**
**DE-A-2 634 511**
**EP-A-0 010 287**
**DE-A-2 739 352**

(73) Patentinhaber: **BASF Aktiengesellschaft, Carl-Bosch-Strasse 38, D-6700 Ludwigshafen (DE)**

(72) Erfinder: **Ammermann, Eberhard, Dr., Sachsenstrasse 3, D-6700 Ludwigshafen (DE)**
Erfinder: **Rentzea, Costin, Dr., Neuenheimer Landstrasse 72, D-6900 Heidelberg (DE)**
Erfinder: **Jung, Johann, Dr. Dipl.-Landwirt, Hardenburgstrasse 19, D-6703 Limburgerhof (DE)**

**EP 0 019 762 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Triazolylderivate, Verfahren zu ihrer Herstellung sowie diese enthaltende Mittel zur Beeinflussung des Pflanzenwachstums

Die vorliegende Erfindung betrifft neue Triazolylderivate, Verfahren zu ihrer Herstellung, Mittel zur Regulierung des Pflanzenwachstums, die diese Verbindungen enthalten, und Verfahren zur Regulierung des Pflanzenwachstums mit diesen Verbindungen.

Es ist bekannt, das 2-Chlorethyl-trimethylammoniumchlorid (Chlorcholinchlorid, CCC) (J. Biol. Chem., 235 475 (1960)) zur Beeinflussung des Pflanzenwachstums zu verwenden. Mit seiner Hilfe lässt sich z.B. eine Hemmung des Längenwachstums bei einigen Getreidearten und eine Hemmung des vegetativen Wachstums bei einigen anderen Kulturpflanzen erreichen. Die Wirkung dieses Stoffes ist jedoch, insbesondere bei niedrigen Aufwandmengen, nicht immer ausreichend und genügt den Anforderungen der Praxis nicht.

Weiterhin ist es bekannt, 1-(4'-Bromphenyl)-1-allyloxy-2-(1'', 2'', 4''-triazolyl-(1''))-ethan zur Regulierung des Pflanzenwachstums von Raps, Weizen, Hafer, Roggen und Gerste zu verwenden (DE-OS 26 50 831). Dessen Wirkung ist jedoch, vor allem bei niedrigen Aufwandmengen nicht immer befriedigend.

Es ist ferner bekannt, 3,3-Dimethyl-2-(1,2,4-triazolyl-(1))-1-(4-chlorbenzoyl)-butan zur Beeinflussung des Pflanzenwachstums zu verwenden (DE-OS 27 39 352).

Aus der europäischen Patentanmeldung 0 000 940 sind Gamma-Azolylakohole bekannt, die zur Regulierung des Pflanzenwachstums geeignet sind. Aus der deutschen Offenlegungsschrift 26 34 511 sind Beta-Azolylketone bekannt, die eine fungizide Wirkung haben. Die neuen Verbindungen gemäss der vorliegenden Erfindung werden hierdurch nicht nahegelegt, da andere bekannte Verbindungen mit ähnlicher chemischer Struktur überraschend eine wesentlich schlechtere Wirkung zeigen.

Es wurde nun gefunden, dass Verbindungen der Formel

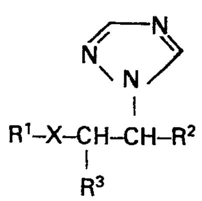

(I)

in der

$R^1$ und $R^2$ unabhängig voneinander Alkyl mit 1 bis 6 C-Atomen, Naphthyl oder Phenyl bedeuten, wobei der Phenylrest durch Fluor, Chlor, Brom, Alkyl oder Alkoxy mit 1 bis 4 C-Atomen substituiert sein kann und $R^3$ Alkyl mit 1 bis 4 C-Atomen bedeutet und

X für C=O oder H–C–$OR^4$ steht, worin $R^4$ ein Wasserstoffatom, einen Alkylrest mit 1 bis 4 C-Atomen, einen Allylrest, einen gegenbenenfalls durch Chlor oder Brom substituierten Benzylrest, einen CO–$R^5$-Rest oder einen CO–$NHR^5$-Rest bedeutet, in dem $R^5$ für einen Alkylrest mit 1 bis 4 C-Atomen oder für einen gegenbenenfalls durch Chlor, Brom oder Alkyl mit 1 bis 4 C-Atomen substituierten Phenylrest steht sowie deren Salze und Metallkomplexe ausgezeichnet zur Beeinflussung des Pflanzenwachstums geeignet sind und eine sehr gute Pflanzenverträglichkeit zeigen.

Bevorzugt sind 4-Chlorphenyl-(3'-(1'',2'',4''-triazol-1''-yl)-4',4'-dimethylpentyl-2')-keton und Methyl-(1-(4'-chlorphenyl)-2,4,4-trimethyl-3-(1'',2'',4''-triazol-1''-yl)-pentyl-1)-ether.

Die neuen Verbindungen der Formel (I) besitzen zwei bzw. drei Asymmetriezentren. Aus den bei der Synthese üblicherweise anfallenden Diastereomerengemischen kann man mit bekannten Methoden einheitliche Diastereomerenpaare bzw. einheitliche Enantiomere erhalten. Diese werden von der vorliegenden Erfindung ebenfalls umfasst. Als Mittel zur Beeinflussung des Pflanzenwachstums kann man sowohl die einheitlichen Diastereomeren bzw. Enantiomeren wie auch deren bei der Synthese anfallenden Gemische verwenden. Bevorzugt werden die letzteren verwendet.

Es wurde weiter gefunden, dass man Triazolylderivate der Formel (I) dadurch herstellen kann, dass man

a) 1,2,4-Triazol mit α,β-ungesättigten Ketonen der Formel (II)

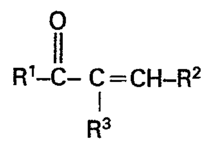

(II)

in welcher $R^1$, $R^2$ und $R^3$ die oben angegebenen Bedeutungen haben, gegebenenfalls in Gegenwart eines Lösungs- oder Verdünnungsmittels und gegebenenfalls in Gegenwart eines basischen Katalysators umsetzt.

Die α,β-ungesättigten Ketone der Formel (II) sind teilweise bekannt wie beispielsweise das 1,2,3-Triphenylpropen-2-on-1, für das in der DE-OS 26 59 293 eine günstige Synthese beschrieben wird. Analog oder nach den bekannten Methoden der gezielten Aldolkondensation (vgl. z.B. T. Mukaiyama, K. Banno und K. Narasaka, J. Am. Chem. Soc. 96 (1974), S. 7503) können die entsprechenden α,β-ungesättigten Ketone der Formel (II) hergestellt werden.

Die Umsetzung der α,β-ungesättigten Ketone mit 1,2,4-Triazol nach dem oben beschriebenen Verfahren zu den β-Triazolylketonen der Formel (III)

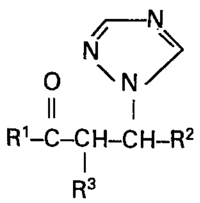

(III)

in der $R^1$, $R^2$ und $R^3$ die oben genannten Bedeutungen haben, wird zweckmässig ohne Verdünnungsmittel oder in einem indifferenten Verdünnungsmittel wie beispielsweise Methanol, Ethanol, Isopropanol, Tetrahydrofuran, Dioxan, Toluol, Acetonitril oder Dimethylformamid bei Temperaturen von 0 bis 100 °C, vorzugweise +20 bis +60 °C durchgeführt.

Es ist zweckmässig, einen basischen Katalysator wie z.B. Natriumhydroxid, Kaliumhydroxid, Triethylamin oder N,N-Dimethylcyclohexylamin dem Reaktionsgemisch zuzugeben.

Es wurde weiter gefunden, dass man Triazolylderivate der Formel (I) auch erhält, wenn man
b) β-Triazolylketone der Formel III katalytisch mit Wasserstoff oder mit Hydriden zu den γ-Triazolylalkoholen der Formel (IV)

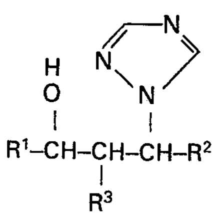

(IV)

in der $R^1$, $R^2$ und $R^3$ die oben genannten Bedeutungen haben, gegebenenfalls in Gegenwart eines Lösungs- oder Verdünnungsmittels reduziert
und
γ-Triazolalkohole der Formel (IV)
α) mit einem Alkylierungsmittel der Formel (V) umsetzt

$$Hal–R^4 \qquad (V)$$

worin Hal für Chlor, Brom oder Jod steht und $R^4$ die oben angegebene Bedeutung mit Ausnahme des Wasserstoffs hat oder
β) mit einem Säurehalogenid der Formel (IV) umsetzt

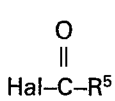

(VI)

worin Hal für Chlor oder Brom und $R^5$ für die oben genannten Reste steht oder
γ) mit einem Isocyanat der Formel (VII)

$$O{=}C{=}N–R^5 \qquad (VII)$$

worin $R^5$ die oben angegebene Bedeutung hat, umsetzt, wobei die Umsetzungen α, β und γ jeweils gegebenenfalls in Gegenwart eines Lösungs- oder Verdünnungsmittels bei Temperaturen zwischen +10 und +100 °C gegebenenfalls in Gegenwart anorganischer oder organischer Basen sowie gegebenenfalls in Gegenwart eines Reaktionsbeschleunigers durchgeführt werden und die gemäss Verfahren a) bis c) erhaltenen Verbindungen gegebenenfalls mit Säuren oder Metallsalzen umsetzt.

Geeignete Lösungs- oder Verdünnungsmittel für das Verfahren b) sind beispielsweise Wasser, Methanol, Ethanol, Diethylether, Tetrahydrofuran, Dioxan oder Toluol.

Für die als Reduktionsmittel verwendbaren Hydride seien beispielsweise Natriumborhydrid oder Lithiumaluminiumhydrid genannt.

Zur katalytischen Hydrierung verwendet man Platin- oder Palladiumkatalysatoren auf inerten Trägern und hydriert bei Drucken von 2 bis 50 bar bis keine Wasserstoffaufnahme mehr erfolgt.

Für die unter c) genannten Verfahren eignen sich folgende Lösungs- oder Verdünnungsmittel: Diethylether, Tetrahydrofuran, Dioxan, Cyclohexan, Methylenchlorid, Chloroform, Toluol oder Dimethylformamid.

Für die erwähnten anorganischen oder organischen Basen seien beispielsweise genannt: Alkali- und Erdalkalihydroxide wie Natriumhydroxid, Kaliumhydroxid, Calziumhydroxid, Alkalicarbonate wie Kalium- oder Natriumcarbonat, Alkalihydride wie Natriumhydrid, Alkali- oder Erdalkalialkoholate wie Natriummethylat, Magnesiummethylat oder Natriumisopropylat oder tertiäre Amine wie Trimethylamin, Triethylamin, N,N-Dimethylanilin, N,N-Dicyclohexylamin, N-Methylpiperidin oder Pyridin.

Mit geeigneten Basen wie z.B. Alkalihydrid wie Natriumhydrid oder Alkali- oder Erdalkalialkoholaten wie Natriummethylat können die γ-Triazolylalkohole der Formel (IV) auch in einer ersten Umsetzung zunächst in ihre Alkoholat-Salze überführt werden und dann als solche zur Reaktion gebracht werden.

Als Reaktionsbeschleuniger für die unter c) genannten Verfahren kommen vorzugsweise Metallhalogenide wie Kaliumjodid, Kronenether, quartäre Ammoniumverbindungen wie Tetrabutylammoniumjodid oder Kombinationen dieser Reaktionsbeschleuniger in Frage.

Die so erhaltenen Verbindungen der Formel (I) werden nach üblichen Methoden isoliert. Im allgemeinen bedürfen sie keiner weiteren Reinigung, sie können aber nach bekannten Methoden wie Umkristallisation, Extraktion, Destillation oder Chromatographie weiter gereinigt werden.

Die neuen Wirkstoffe der Formel (I) können auch in Form ihrer Salze, insbesondere in Form der für Pflanzen verträglichen Salze, wie Säureadditionssalze z.B. Hydrochloride, Hydrooxalate oder Hydronitrate verwendet werden.

Falls gewünscht, können die Triazolylderivate der Formel (I) auch nach bekannten Verfahren in ihre Metallkomplexe, insbesondere in die für Pflanzen verträglichen Metallkomplexe, überführt werden. Solche Metallkomplexe erhält man beispielsweise durch Umsetzung eines Triazolylderivates der Formel (I) mit einem Metallsalz wie einem Salz von Kobalt, Eisen, Nickel, Kupfer, Zink oder Mangan, z.B. Kobaltchlorid, Eisen-II-sulfat, Nickelnitrat, Kupfer-II-sulfat, Zinkchlorid, Kupfer-II-chlorid oder Mangan-II-sulfat.

Die neuen Verbindungen können praktisch alle Entwicklungsstadien einer Pflanze verschiedenartig beeinflussen und werden deshalb als Mittel zur Bekämpfung des Pflanzenwachstums eingesetzt. Ihre Wirkungsvielfalt hängt vor allem

a) von der Pflanzenart und -sorte,

b) von dem Zeitpunkt derApplikation, bezogen auf das Entwicklungsstadium der Pflanze, und von der Jahreszeit,

c) von dem Applikationsort und -verfahren (Samenbeize, Bodenbehandlung oder Blattapplikation)

d) von den geoklimatischen Faktoren, z.B. Sonnenscheindauer, Durchschnittstemperatur, Niederschlagsmenge,

e) von der Bodenbeschaffenheit (einschliesslich Düngung),

f) von der Formulierung bzw. Anwendungsform des Wirkstoffs und schliesslich

g) von den angewendeten Konzentrationen der aktiven Substanz.

Aus der Reihe der verschiedenartigen Anwendungsmöglichkeiten der Pflanzenwachstumsregulatoren im Pflanzenbau, in der Landwirtschaft und im Gartenbau, werden einige nachstehend erwähnt.

A. Mit den erfindungsgemäss verwendbaren Verbindungen lässt sich das vegetative Wachstum der Pflanzen stark hemmen, was sich insbesondere in einer Reduzierung des Längenwachstums äussert. Die behandelten Pflanzen weisen demgemäss einen gedrungenen Wuchs auf; ausserdem ist eine dunklere Blattfärbung zu beobachten.

Als vorteilhaft für die Praxis erweist sich z.B. die Verringerung des Grasbewuchses an Strassenrändern, Kanalböschungen und auf Rasenflächen wie Park-, Sport- und Obstanlagen, Zierrasen und Flugplätzen, so dass der arbeits- und kostenaufwendige Rasenschnitt reduziert werden kann.

Von wirtschaftlichem Interesse ist auch die Erhöhung der Standfestigkeit von lageranfälligen Kulturen wie Getreide, Mais und Soja. Die dabei verursachte Halmverkürzung und Halmverstärkung verringert oder beseitigt die Gefahr des «Lagerns» (des Umknickens) von Pflanzen unter ungünstigen Witterungsbedingungen vor der Ernte.

Wichtig ist auch die Anwendung zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.

Durch Anwendung der Mittel kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.

Mit den Mitteln lässt sich beispielsweise bei Winterraps auch die Frostresistenz erheblich erhöhen. Dabei werden einerseits das Längenwachstum und die Entwicklung einer üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generative Phase neigen. Auch bei anderen Kulturen, z.B. Wintergetreide ist es vorteilhaft, wenn die Bestände durch Behandlung mit erfindungsgemässen Verbindungen im Herbst zwar gut bestockt, aber nicht zu üppig in den Winter hineingehen. Dadurch kann der erhöhten Frostempfindlichkeit und – wegen der relativ geringen Blatt- bzw. Pflanzemasse – dem Befall mit verschiedenen, insbesondere pilzlichen, Krankheiten vorgebeugt werden. Die Hemmung des vegetativen Wachstums ermöglicht ausserdem bei vielen Kulturpflanzen eine dichtere Bepflanzung, so dass ein Mehrertrag bezogen auf die Bodenfläche erzielt werden kann.

B. Mit den neuen Wirkstoffen lassen sich Mehrerträge sowohl an Pflanzenteilen als auch an Pflanzeninhaltsstoffen erzielen. So ist es beispielsweise möglich, das Wachstum grösserer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zu vermehrtem Latexfluss zu stimulieren.

Dabei können die neuen Stoffe Ertragssteigerungen durch Eingriffe in den pflanzlichen Stoffwechsel bzw. durch Förderung oder Hemmung des vegetativen und/oder des generativen Wachstums verursachen.

C. Mit den Mitteln lassen sich schliesslich sowohl eine Verkürzung bzw. Verlängerung der Wachstumsstadien als auch eine Beschleunigung bzw. Verzögerung der Reife der geernteten Pflanzenteile vor oder nach der Ernte erreichen.

Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heisst die Förderung der Ausbildung von Trenngewebe zwischen Frucht- bzw. Blatt und Sprossteil der Pflanze ist auch für ein gut kontrollierbares Entblättern der Bäume wesentlich.

Die Wirkung der neuen Verbindungen ist besser als bei bekannten Wachstumsregulatoren. Die Wirkung zeigt sich sowohl bei Monokotylen, z.B. Getriede wie Weizen, Gerste, Roggen, Hafer und Reis oder Mais als auch bei Dikotylen (z.B. Sonnenblumen, Tomaten, Soja, Reben, Baumwolle und vor allem Raps) und verschiedene Zierpflanzen wie Chrysanthemen, Poinsettien und Hibiskus.

Die neuen Triazolylderivate können den Kulturpflanzen sowohl vom Samen her (als Saatgutbeizmittel) als auch über den Boden, d.h. durch die Wurzel sowie durch Spritzung über das Blatt zugeführt werden.

Infolge der hohen Pflanzenverträglichkeit kann die Aufwandmenge stark variiert werden.

Bei der Saatgutbehandlung werden im allgemeinen Wirkstoffmengen von 0,001 bis 50 g je Kilogramm Saatgut, vorzugsweise 0,01 bis 10 g benötigt.

Für die Blatt- und Bodenbehandlung sind im allgemeinen Gaben von 0,01 bis 12 kg/ha bevorzugt 0,25 bis 3 kg/ha als ausreichend zu betrachten.

Die erfindungsgemässen Mittel können in Form üblicher Formulierungen angewendet werden wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmässige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Ethanolamin), Dimethylformamid und Wasser; feste Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate) und Dispergiermittel wie Lignin, Sulfitablaugen und Methylcellulose.

Die Formulierungen enthalten im allgemeinen zwischen 0,1 und 95 Gewichtsprozent Wirkstoff, vorzugsweise zwischen 0,5 und 90.

Die Formulierungen bzw. die daraus hergestellten gebrauchsfertigen Zubereitungen wie Lösungen, Emulsionen, Suspensionen, Pulver, Stäube, Pasten oder Granulate werden in bekannter Weise angewendet, beispielsweise im Vorauflaufverfahren, im Nachauflaufverfahren oder als Beizmittel.

Die erfindungsgemässen Mittel können in diesen Aufwendungsformen auch zusammen mit anderen Wirkstoffen vorliegen, wie z.B. Herbiziden, Insektiziden, Wachstumsregulatoren und Fungiziden oder auch mit Düngemitteln vermischt und ausgebracht werden. Beim Vermischen mit Wachstumsregulatoren erhält man dabei in vielen Fällen eine Vergrösserung des Wirkungsspektrums. Bei einer Anzahl solcher Wachstumsregulatormischungen treten auch synergistische Effekte auf, d.h. die Wirksamkeit des Kombinationsproduktes ist grösser als die addierten Wirksamkeiten der Einzelkomponenten.

Die folgenden Beispiele erläutern die Herstellung der neuen Verbindungen:

Beispiel 1

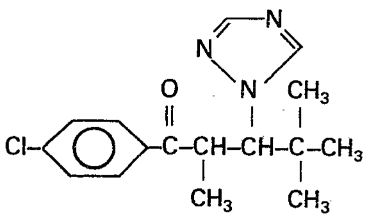

(Nr. 1)

31 g 1-(4'-Chlorphenyl)-2,4,4-trimethylpenten-2-on-1 werden in 250 ml Ethanol mit 27 g 1,2,4-Triazol und 0,2 g Kaliumhydroxid vier Tage lang bei 60 °C gerührt. Dann wird das Reaktionsgemisch eingeengt, in Methylenchlorid aufgenommen und viermal mit Wasser gewaschen. Die organische Phase wird getrocknet, eingeengt und an Kieselgel mit Cyclohexan-Essigester-(1 bis 50%)-Gemischen getrennt. Man erhält so 12 g 4-Chlorphenyl-(3'-(1'',2'',4''-triazol- 1''-yl)-4',4'-dimethylpentyl -2'-)-keton als hellgelbes Öl.$n_D^{20}$ = 1,5172

| | C | H | N | Cl |
|---|---|---|---|---|
| ber. | 62,8 | 6,6 | 13,7 | 11,6 |
| gef. | 62,7 | 6,2 | 13,2 | 11,2 |

Beispiel 2

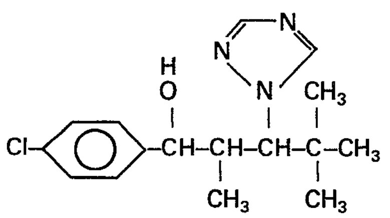

(Nr.2)

14 g 4-Chlorphenyl-(3'-(1'',2'',4''triazol-1''-yl)-4',4'-dimethylpentyl-2'-)-keton werden in 250 ml abs. Methanol gelöst und bei 0 °C portionenweise mit 2,75 g Natriumborhydrid versetzt. Nach dem Rühren über Nacht wird die Mischung mit konz. Salzsäure angesäuert, anschliessend mit Ammoniak neutralisiert und das Reaktionsgemisch zur Trockne eingeengt. Der Rückstand wird in Methylenchlorid aufgenommen und dreimal mit Wasser gewaschen. Nach dem Trocknen und Einengen der organischen Phase verbleiben 11 g 1-(4'-Chlorphenyl)-2,4,4-trimethyl-3-(1'',2'',4''-triazol-1''-yl)-pentyl-1-alkohol als klares Öl ($n_D^{20}$ = 1,5350).

| | C | H | N | Cl |
|---|---|---|---|---|
| ber. | 62,6 | 6,9 | 13,7 | 11,5 |
| gef. | 62,1 | 6,6 | 13,3 | 10,9 |

Beispiel 3

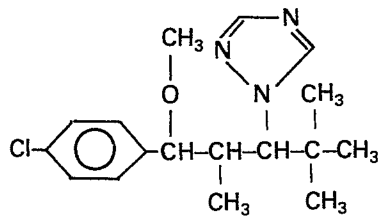

(Nr. 3)

In 150 ml abs. Tetrahydrofuran (THF) werden 1,7 g Hatriumhydrid suspendiert und bei 40 °C langsam mit einer Lösung von 10 g 1-(4'-Chlorphenyl)-2,4,4-trimethyl-3-(1'',2'',4''-triazol-1''-yl)-pentyl-1-alkohol in 50 ml abs. THF versetzt. Nach zweistündigem Rühren bei 50 °C wird auf Raumtemperatur abgekühlt und eine Lösung von 7,1 g Methyljodid in 10 ml abs. THF zugetropft. Nach dem Rühren über Nacht wird das überschüssige Natriumhydrid mit Wasser zersetzt. Das Reaktionsgemisch wird mit Schwefelsäure neutralisiert und eingeengt. Der Rückstand wird in Wasser aufgenommen und zweimal mit Methylenchlorid extrahiert. Die Lösung wird getrocknet und das Lösungsmittel abgedampft. Es verbleiben 9 g Methyl-(1-(4'-chlorphenyl)-2,4,4-trimethyl-3(1'',2'', 4''-triazol-1''-yl)-pentyl-1)-ether als farbloses Öl.

| | C | H | N | Cl |
|---|---|---|---|---|
| ber. | 63,4 | 7,5 | 13,0 | 11,0 |
| gef. | 63,7 | 7,9 | 12,6 | 10,7 |

Beispiel 4

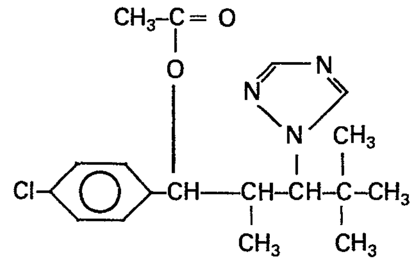

(Nr. 4)

In 200 ml abs. THF werden 15 g 1-(4'-Chlorphenyl)-2,4,4-trimethyl-3-(1'',2'',4''-triazol-1''-yl-)pentyl-1-alkohol, 1 g Imidazol und 8,5 ml Triethylamin gelöst. Unter Rühren werden 5,6 ml Acetanhydrid zugetropft und anschliessend wird für 2 Stunden bei Rückflusstemperatur gekocht und gerührt. Nach dem Abkühlen wird eingeengt, der Rückstand in Methylenchlorid aufgenommen und dreimal mit Wasser gewaschen. Die organische Phase wird nach dem Trocknen eingeengt und ans Kieselgel mit Methylenchlorid als Elutionsmittel gereinigt. Man erhält so 8 g Essigsäure-1-(4'-chlorphenyl)-2,4,4-trimethyl-3 (1'',2'',4''triazol-1''-yl)-pentyl-1-ester als farbloses Öl ($n_D^{20}$ = 1,5071).

| | C | H | N | Cl |
|---|---|---|---|---|
| ber. | 61,9 | 6,6 | 12,0 | 10,1 |
| gef. | 61,7 | 7,0 | 11,8 | 9,8 |

Analog wurden die folgenden Triazolylderivate der Formel (I) hergestellt, die durch UR- und NMR-Spektroskopie sowie durch Elementaranalyse charakterisiert wurden.

Tabelle

| Nr. | $R^1$ | X | $R^2$ | $R^3$ | Fp( °C) | $n_D^{20}$ |
|---|---|---|---|---|---|---|
| 5 | $C_6H_5$ | HCOH | $C_6H_5-$ | $CH_3-$ | 136–138 | – |
| 6 | $C_6H_5-$ | C=O | $2-Cl-C_6H_4-$ | $CH_3$ – | 147–150 | – |
| 7 | $C_6H_5-$ | C=O | $C_6H_5-$ | $CH_3-$ | 111–114 | – |
| 8 | $C_6H_5-$ | C=O | 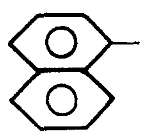 | $CH_3-$ | 179–181 | – |
| 9 | $C_6H_5-$ | C=O | $n-C_3H_7-$ | $CH_3-$ | Öl | – |
| 10 | $C_6H_5$ – | C=O | tert.–$C_4H_9-$ | $CH_3-$ | Öl | – |
| 11 | $4-F-C_6H_4-$ | C=O | tert.–$C_4H_9-$ | $CH_3-$ | | 1,518 |
| 12 | $C_6H_5-$ | C=O | tert.–$C_4H_9-$ | $n-C_4H_9-$ | | 1,5022 |
| 13 | $C_6H_5-$ | C=O | $i-C_3H_7-$ | $-CH_3$ | Öl | – |
| 14 | $4-Cl-C_6H_4-$ | C=O | $i-C_3H_7-$ | $-CH_3$ | ÖL | – |
| 15 | $4-Cl-C_6H_4-$ | C=O | $n-C_3H_7-$ | $-CH_3$ | Öl | – |
| 16 | $4-Cl-C_6H_4-$ | $HC-OCH_2-CH_3$ | tert.–$C_4H_9-$ | $-CH_3$ | | |
| 17 | $4-Cl-C_6H_4-$ | $HC-OCOCH_2-CH_3$ | tert.–$C_4H_9-$ | $-CH_3$ | | 1,5009 |
| 18 | $4-Cl\ -C_6H_4-$ | $HCOCH_2C_6H_5$ | tert.–$C_4H_9-$ | $CH_3-$ | | |
| 19 | $4-Cl-C_6H_4-$ | $HCO(CO)C_6H_5$ | tert.–$C_4H_9-$ | $CH_3-$ | | |
| 20 | $4-Cl-C_6H_4-$ | $HCO(CO)NHC_6H_5$ | tert.–$C_4H_9-$ | $CH_3-$ | | |
| 21 | $4-Cl-C_6H_4$ | $HCO-CH_2-CH=CH_2$ | tert.–$C_4H_9-$ | $CH_3-$ | | |

In den nachfolgenden Beispielen wird die Wirkung der erfindungsgemäss verwendbaren Stoffe als Pflanzenwachstumsregulatoren dargestellt.

Zur Bestimmung der wachstumsregulierenden Eigenschaft der Prüfsubstanzen wurden Testpflanzen auf ausreichend mit Nährstoffen versorgtem Boden in Kunststoffgefässen von ca. 12,5 cm Durchmesser angezogen.

Im Vorauflaufverfahren wurden die Testsubstanzen in wässriger Aufbereitung am Tage der Einsaat auf das Saatbett gegossen. Im Nachauflaufverfahren wurden die zu prüfenden Substanzen in wässriger Aufbereitung auf die Pflanzen gesprüht. Die beobachtete wachstumsregulierende Wirkung wurde bei Versuchsende durch Wuchshöhenmessung belegt. Die so gewonnenen Messwerte wurden zur Wuchshöhe der unbehandelten Pflanzen in Relation gesetzt. Als Vergleichssubstanzen dienten die folgenden bekannten Wirkstoffe.

Die Einzeldaten sind den folgenden Tabellen zu entnehmen.

Kurzbezeichnung der Wirkstoffe

CCC

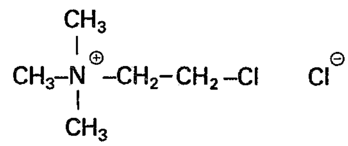

Bekannt aus
DE-OS 26 50 831

A

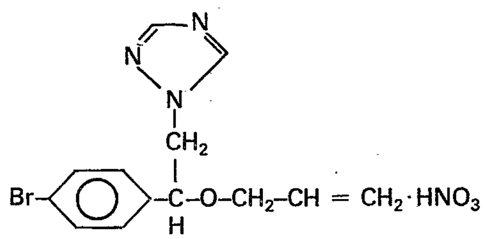

Bekannt aus
DE-OS 27 39 352

B

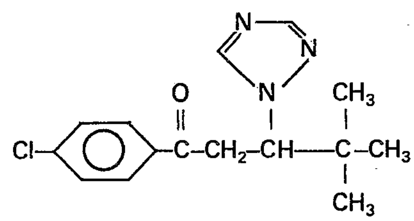

Tabelle 1
Vorauflaufverfahren – Sommerweizen «Kolibri»
Versuchsdauer: 14 Tage

| Wirkstoff | Konzentration mg WS/Gef.* | Wuchshöhen cm | % |
|---|---|---|---|
| unbehandelt | – | 26,1 | 100 |
| CCC | 3 | 18,5 | 70,9 |
| | 12 | 17,5 | 67,0 |
| 1 | 3 | 15,5 | 59,4 |
| | 12 | 11,0 | 42,1 |
| 13 | 3 | 15,0 | 57,5 |
| | 12 | 8,0 | 30,7 |
| 14 | 3 | 15,0 | 57,5 |
| | 12 | 10,0 | 38,3 |

*WS = Wirkstoff

Tabelle 2
Nachauflaufverfahren – Sommerweizen «Kolibri»
Versuchsdauer: 14 Tage

| Wirkstoff | Konzentration mg WS/Gef. | Wuchshöhen cm | % |
|---|---|---|---|
| unbehandelt | – | 26,3 | 100 |
| B | 1,5 | 24,0 | 91,3 |
| | 6 | 23,5 | 89,4 |
| 1 | 1,5 | 20,5 | 77,9 |
| | 6 | 20,5 | 77,9 |
| 13 | 1,5 | 22,0 | 83,7 |
| | 6 | 21,0 | 79,8 |
| 14 | 1,5 | 22,5 | 85,6 |
| | 6 | 22,0 | 83,7 |

Tabelle 3
Vorauflaufverfahren – Sommergerste «Union»
Versuchsdauer: 15 Tage

| Wirkstoff | Konzentration mg WS/Gef. | Wuchshöhen cm | % |
|---|---|---|---|
| unbehandelt | – | 26,9 | 100 |
| CCC | 3 | 21,5 | 79,9 |
| | 12 | 19,5 | 72,5 |
| A | 3 | 25,5 | 94,8 |
| | 12 | 22,0 | 81,8 |
| B | 3 | 23,0 | 85,5 |
| | 12 | 18,0 | 66,9 |
| 13 | 3 | 21,5 | 79,9 |
| | 12 | 13,0 | 48,3 |
| 14 | 3 | 21,0 | 78,1 |
| | 12 | 16,0 | 59,5 |

Tabelle 4
Nachauflaufverfahren – Sommergerste «Union»
Versuchsdauer: 15 Tage

| Wirkstoff | Konzentration mg WS/Gef. | Wuchshöhen cm | % |
|---|---|---|---|
| unbehandelt | – | 27,7 | 100 |
| CCC | 1,5 | 26,0 | 93,9 |
| | 6 | 25,0 | 90,3 |
| A | 1,5 | 27,0 | 97,5 |
| | 6 | 25,0 | 90,3 |
| B | 1,5 | 27,0 | 97,5 |
| | 6 | 26,5 | 95,7 |
| 1 | 1,5 | 25,0 | 90,3 |
| | 6 | 22,0 | 79,4 |

Tabelle 5
Vorauflaufverfahren – Hafer «Flämingkrone»
Versuchsdauer: 17 Tage

| Wirkstoff | Konzentration mg WS/Gef. | Wuchshöhen cm | % |
|---|---|---|---|
| unbehandelt | – | 26,4 | 100 |
| CCC | 3 | 24,5 | 92,8 |
| | 12 | 23,5 | 89,0 |
| A | 3 | 24,5 | 92,8 |
| | 12 | 21,0 | 79,6 |
| 13 | 3 | 24,0 | 90,0 |
| | 12 | 16,0 | 60,6 |
| 14 | 3 | 23,5 | 89,0 |
| | 12 | 17,0 | 64,4 |

Tabelle 6
Nachauflaufverfahren – Rasen
Versuchsdauer: 63 Tage

| Wirkstoff | Konzentration mg WS/Gef. | Wuchshöhen cm | % |
|---|---|---|---|
| unbehandelt | – | 10,3 | 100 |
| B | 1,5 | 9,0 | 87,4 |
| | 6 | 8,0 | 77,7 |
| 2 | 1,5 | 7,0 | 68,0 |
| | 6 | 5,0 | 48,5 |
| 13 | 1,5 | 9,0 | 87,4 |
| | 6 | 7,0 | 68,0 |
| 14 | 1,5 | 9,0 | 87,4 |
| | 6 | 7,0 | 68,0 |

Tabelle 7
Nachauflaufverfahren – Soja «SRF 450»
Versuchsdauer: 28 Tage

| Wirkstoff | Konzentration mg WS/Gef. | Wuchshöhen cm | % |
|---|---|---|---|
| unbehandelt | – | 25,6 | 100 |
| CCC | 1,5 | 24,0 | 93,8 |
| | 6 | 21,0 | 82,0 |
| A | 1,5 | 25,5 | 99,6 |
| | 6 | 23,5 | 91,8 |
| B | 1,5 | 25,0 | 97,7 |
| | 6 | 24,5 | 95,7 |
| 1 | 1,5 | 20,5 | 80,1 |
| | 6 | 15,0 | 58,6 |

Gleichlaufend zur Reduzierung des Längenwachstums stieg die Farbintensität der Blätter an. Der erhöhte Chlorophyllgehalt lässt eine ebenfalls erhöhte Photosyntheserate und damit eine erhöhte Ertragsbildung erwarten.

Tabelle 8
Nachauflaufverfahren – Soja «SRF 450»
Versuchsdauer: 29 Tage

| Wirkstoff | Konzentration mg WS/Gef. | Wuchshöhen cm | % |
|---|---|---|---|
| unbehandelt | – | 20,8 | 100 |
| CCC | 1,5 | 20,5 | 98,6 |
| | 6 | 19,5 | 93,8 |
| B | 1,5 | 20,0 | 96,2 |
| | 6 | 20,0 | 96,2 |
| 3 | 1,5 | 16,5 | 79,3 |
| | 6 | 11,5 | 55,3 |

Tabelle 9
Nachauflaufverfahren – Soja «SRF 400»
Versuchsdauer: 40 Tage

| Wirkstoff | Konzentration mg WS/Gef. | Wuchshöhe cm | % |
|---|---|---|---|
| unbehandelt | – | 26,3 | 100 |
| CCC | 1,5 | 25,0 | 95,1 |
| | 6 | 24,0 | 91,3 |
| B | 1,5 | 24,0 | 91,3 |
| | 6 | 24,0 | 91,3 |
| 7 | 1,5 | 24,0 | 91,3 |
| | 6 | 23,5 | 89,4 |
| 8 | 1,5 | 19,5 | 74,1 |
| | 6 | 19,0 | 72,2 |

Tabelle 10
Vorauflaufverfahren – Sommerraps «Cosa»
Versuchsdauer: 22 Tage

| Wirkstoff | Konzentration mg WS/Gef. | Wuchshöhen cm | % |
|---|---|---|---|
| unbehandelt | – | 21,1 | 100 |
| CCC | 3 | 19,0 | 90,1 |
| | 12 | 18,5 | 87,7 |
| A | 3 | 16,0 | 75.8 |
| | 12 | 10,5 | 49,8 |
| B | 3 | 15,5 | 73,5 |
| | 12 | 11,5 | 54,5 |
| 1 | 3 | 14,0 | 66,4 |
| | 12 | 7,0 | 33,2 |

Tabelle 11
Nachauflaufverfahren – Sommerraps «Cosa»
Versuchsdauer: 21 Tage

| Wirkstoff | Konzentration mg WS/Gef. | Wuchshöhen cm | % |
|---|---|---|---|
| unbehandelt | – | 21,3 | 100 |
| CCC | 1,5 | 19,5 | 91,6 |
| | 6 | 19,0 | 89,2 |
| B | 1,5 | 19,5 | 91,6 |
| | 6 | 18,0 | 84,5 |
| 1 | 1,5 | 15,0 | 70,4 |
| | 6 | 8,0 | 56,3 |
| 2 | 1,5 | 19,0 | 89,2 |
| | 6 | 16,5 | 77,5 |
| 11 | 1,5 | 20,0 | 93,9 |
| | 6 | 15,0 | 70,4 |

Beispiel A

Man vermischt 90 Gewichtsteile der Verbindung 1 mit 10 Gewichtsteilen N-Methyl-α-pyrrolidon und erhält eine Lösung, die zur Anwendung in Form kleinster Tropfen geeignet ist.

Beispiel B

20 Gewichtsteile der Verbindung 3 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Äthylenoxid an 1 Mol Isooctylphenol und 10 Gewichtsteilen des Anlagerungsproduktes von 40 Mol Äthylenoxid an 1 Mol Ricinusöl besteht. Durch Eingiessen und feines Verteilen der Lösung in 100 000 Gewichtsteilen Wasser erhält man eine wässrige Dispersion, die 0,02 Gew.-% des Wirkstoffs enthält.

Beispiel C

20 Gewichtsteile der Verbindung 1 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Äthylenoxid an 1 Mol Ricinusöl besteht. Durch Eingiessen und feines Verteilen der Lösung in 100 000 Gewichtsteilen Wasser erhält man eine wässrige Dispersion, die 0,02 Gew.-% des Wirkstoffs enthält.

Beispiel D

20 Gewichtsteile des Wirkstoffs 3 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen

der Mischung in 20 000 Gewichtsteilen Wasser erhält man eine Spritzbrühe, die 0,1 Gewichtsprozent des Wirkstoffs enthält.

Beispiel E

3 Gewichtsteile der Verbindung 3 werden mit 97 Gewichtsteilen feinteiligem Kaolin innig vermischt. Man erhält auf diese Weise ein Stäubemittel, das 3 Gew.-% des Wirkstoffs enthält.

Beispiel F

30 Gewichtsteile der Verbindung 4 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

**Patentansprüche für die Vertragsstaaten BE, CH, DE, FR, GB, IT, LI, LU, NL und SE**

1. Triazolylderivate der allgemeinen Formel (I)

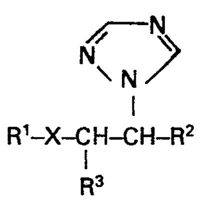

(I)

in der

$R^1$ und $R^2$ unabhängig voneinander Alkyl mit 1 bis 6 C-Atomen, Naphthyl oder Phenyl bedeuten, wobei der Phenylrest durch Fluor, Chlor, Brom, Alkyl oder Alkoxy mit 1 bis 4 C-Atomen substituiert sein kann und

$R^3$ Alkyl mit 1 bis 4 C-Atomen bedeutet und

X für C=O oder H–C–$OR^4$ steht, worin $R^4$ ein Wasserstoffatom, einen Alkylrest mit 1 bis 4 C-Atomen, einen Allylrest, einen gegebenenfalls durch Chlor oder Brom substituierten Benzylrest einen CO–$R^5$-Rest oder einen CO–$NHR^5$-Rest bedeutet, in dem $R^5$ für einen Alkylrest mit 1 bis 4 C-Atomen oder für einen gegebenenfalls durch Chlor, Brom oder Alkyl mit 1 bis 4 C-Atomen substituierten Phenylrest steht,

sowie deren Salze und Metallkomplexe.

2. 4-Chlorphenyl-(3'-(1'',2'',4''-triazol-1''-yl)-4',4'-dimethylpentyl-2'-)-keton

3. Methyl-(1-(4'-chlorphenyl-)-2,4,4-trimethyl-3-(1'',2'',4''-triazol-1''-yl)-pentyl-1)-ether.

4. Verfahren zur Herstellung eines Triazolylderivats gemäss Anspruch 1, dadurch gekennzeichnet, dass man

a) 1,2,4-Triazol mit α,β-ungesättigten Ketonen der Formel (II)

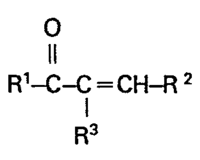

(II)

in welcher $R^1$, $R^2$, und $R^3$ die im Anspruch 1 angegebenen Bedeutungen haben, gegebenenfalls in Gegenwart eines Lösungs- oder Verdünnungsmittels und gegebenenfalls in Gegenwart eines basischen Katalysators umsetzt und gegebenenfalls

b) β-Triazolylketone der Formel (III)

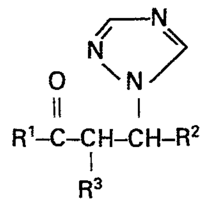

(III)

in der $R^1$, $R^2$ und $R^3$ die im Anspruch 1 genannten Bedeutungen haben, katalytisch mit Wasserstoff oder mit Hydriden zu den Alkoholen der Formel (IV)

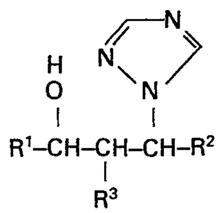

(IV)

gegebenenfalls in Gegenwart eines Lösungs- oder Verdünnungsmittels reduziert

und

c) γ-Triazolylalkohole der Formel (IV), in der $R^1$, $R^2$ und $R^3$ die im Anspruch 1 angegebenen Bedeutungen haben,

α) mit einem Alkylierungsmittel der Formel (V) umsetzt

$$Hal–R^4 \qquad (V)$$

worin Hal für Chlor, Brom oder Jod steht und $R^4$ die im Anspruch 1 angegebene Bedeutung mit Ausnahme von Wasserstoff hat oder

β) mit einem Säurehalogenid der Formel (VI) umsetzt

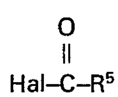

(VI)

worin Hal für Chlor oder Brom und $R^5$ für die im Anspruch 1 genannten Reste steht oder

γ) mit einem Isocyanat der Formel (VII)

$$O=C=N–R^5 \qquad (VII)$$

umsetzt, worin $R^5$ die im Anspruch 1 angegebene Bedeutung hat, wobei die Umsetzungen α, β und γ jeweils gegebenenfalls in Gegenwart eines Lösungs- oder Verdünnungsmittels und gegebenenfalls in Gegenwart anorganischer oder organischer Basen sowie gegebenenfalls in Gegenwart eines Reaktionsbeschleunigers durchgeführt werden und die gemäss Verfahren a) bis c) erhaltenen Verbindungen gegebenenfalls mit Säuren oder Metallsalzen umsetzt.

5. Mittel zur Beeinflussung des Pflanzenwachstums enthaltend ein Triazolylderivat gemäss Anspruch 1, 2 oder 3.

6. Mittel zur Beeinflussung des Pflanzenwachstums enthaltend einen festen oder flüssigen Trägerstoff und ein Triazolylderivat gemäss Anspruch 1.

7. Verfahren zur Herstellung eines Mittels zur Beeinflussung des Pflanzenwachstums gemäss Anspruch 6, dadurch gekennzeichnet, dass man einen festen oder flüssigen Trägerstoff mit einem Triazolylderivat gemäss Anspruch 1 vermischt.

8. Verfahren zur Beeinflussung des Pflanzenwachstums, dadurch gekennzeichnet, dass man die Pflanzen, ihren Samen oder den Boden mit einem Triazolylderivat gemäss Anspruch 1 behandelt.

**Patentansprüche für den Vertragsstaat: AT**

1. Mittel zur Beeinflussung des Pflanzenwachstums, enthaltend ein Triazolylderivat der Formel (I)

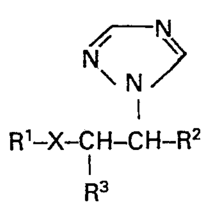

(I)

in der

$R^1$ und $R^2$ unabhängig voneinander Alkyl mit 1 bis 6 C-Atomen, Naphthyl oder Phenyl bedeuten, wobei der Phenylrest durch Fluor, Chlor, Brom, Alkyl oder Alkoxy mit 1 bis 4 C-Atomen substituiert sein kann und

$R^3$ Alkyl mit 1 bis 4 C-Atomen bedeutet und

X für C=O oder H–C–$OR^4$ steht, worin $R^4$ ein Wasserstoffatom, einen Alkylrest mit 1 bis 4 C-Atomen, einen Allylrest, einen gegebenenfalls durch Chlor oder Brom substituierten Benzylrest, einen CO–$R^5$-Rest oder einen CO–$NHR^5$-Rest bedeutet, in dem $R^5$ für einen Alkylrest mit 1 bis 4 C-Atomen oder für einen gegebenenfalls durch Chlor, Brom oder Alkyl mit 1 bis 4 C-Atomen substituierten Phenylrest steht,

sowie deren Salze und Metallkomplexe.

2. Mittel zur Beeinflussung des Pflanzenwachstums gemäss Anspruch 1, dadurch gekennzeichnet, dass das Triazolylderivat, 4-Chlorphenyl-(3'-(1'',2'',4''-triazol-1''-yl)-4',4'-dimethylpentyl-2')-keton ist.

3. Mittel zur Beeinflussung des Pflanzenwachstums gemäss Anspruch 1, dadurch gekennzeichnet, dass das Triazolylderivat Methyl-(1-(4'-chlorphenyl)-2, 4,4-trimethyl-3-(1'',2'',4''-triazol-1''-yl)-pentyl-1)-ether ist.

4. Verfahren zur Herstellung eines Triazolylderivats, wie in Anspruch 1 definiert, dadurch gekennzeichnet, dass man

a) 1,2,4-Triazol mit α, β-ungesättigten Ketonen der Formel (II)

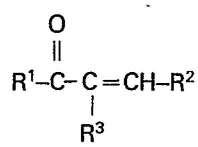

(II)

in welcher $R^1$, $R^2$ und $R^3$ die im Anspruch 1 angegebenen Bedeutungen haben, gegebenenfalls in Gegenwart eines Lösungs- oder Verdünnungsmittels und gegebenenfalls in Gegenwart eines basischen Katalysators umsetzt und gegebenenfalls

b) β-Triazolylketone der Formel (III)

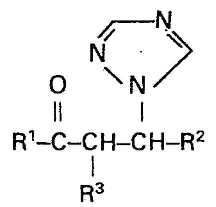

(III)

in der $R^1$, $R^2$ und $R^3$ die im Anspruch 1 genannten Bedeutungen haben, katalytisch mit Wasserstoff oder mit Hydriden zu den Alkoholen der Formel (IV)

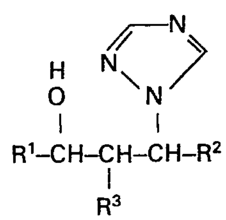

(IV)

gegebenenfalls in Gegenwart eines Lösungs- oder Verdünnungsmittels reduziert

und

c) γ-Triazolylalkohole der Formel IV, in der $R^1$, $R^2$ und $R^3$ die im Anspruch 1 angegebenen Bedeutungen haben,

α) mit einem Alkylierungsmittel der Formel (V) umsetzt

Hal–$R^4$ (V)

worin Hal für Chlor, Brom oder Jod steht und $R^4$ die im Anspruch 1 angegebene Bedeutung mit Ausnahme von Wasserstoff hat oder

β) mit einem Säurehalogenid der Formel (VI) umsetzt

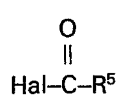

(VI)

worin Hal für Chlor oder Brom und $R^5$ für die im Anspruch 1 genannten Reste steht oder

γ) mit einem Isocyanat der Formel (VII)

O=C=N–$R_5$ (VII)

umsetzt, worin $R^5$ die im Anspruch 1 angegebene Bedeutung hat, wobei die Umsetzungen α, β und γ jeweils gegebenenfalls in Gegenwart eines Lösungs- oder Verdünnungsmittels und gegebenenfalls in Gegenwart anorganischer oder organischer Basen sowie gegebenenfalls in Gegenwart eines Reaktionsbeschleunigers durchgeführt werden und die gemäss Verfahren a) bis c) erhaltenen Verbindungen gegebenenfalls mit Säuren oder Metallsalzen umsetzt.

5. Mittel zur Beeinflussung des Pflanzenwachstums enthaltend einen festen oder flüssigen Trägerstoff und ein gemäss Anspruch 1 definiertes Triazolylderivat.

6. Verfahren zur Herstellung eines Mittels zur Beeinflussung des Pflanzenwachstums, dadurch gekennzeichnet, dass man einen festen oder flüssigen Trägerstoff mit einem gemäss Anspruch 1 definierten Triazolylderivat vermischt.

7. Verfahren zur Beeinflussung des Pflanzenwachstums, dadurch gekennzeichnet, dass man die Pflanzen, ihren Samen oder den Boden mit einem gemäss Anspruch 1 definierten Triazolylderivat behandelt.

**Revendications pour les Etats Contractants BE, CH, DE, FR, GB, IT, LI, LU, NL et SE**

1. Dérivés triazolyliques de formule générale (I)

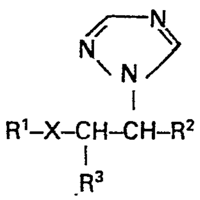

(I)

dans laquelle

$R^1$ et $R^2$ représentent, indépendamment l'un de l'autre, alkyle à 1 à 6 atomes de C, naphtyle, ou phényle, le reste phényle pouvant être substitué par du fluor, chlore, brome, alkyle ou alcoxy à 1 à 4 atomes C et

$R^3$ représente alkyle à 1 à 4 atomes C et

X resprésente C=O ou H–C–OR$^4$, où $R^4$ représente un atome d'hydrogène, un rest alkyle à 1 à 4 atomes C, un reste allyle, un rest benzyle éventuellement substitué par du chlore ou du brome, un reste CO–R$^5$ ou un reste CO–NHR$^5$ dans lequel $R^5$ représente un reste alkyle à 1 à 4 atomes C ou un reste phényle éventuellement substitué par du chlore, brome ou alkyle à 1 à 4 atomes C,

ainsi que leurs sels et complexes métalliques.

2. 4-chlorophényl-(3'-(1'',2'',4''triazol-1''-yl)-4',4'diméthylpentyl-2'-)-cétone.

3. Méthyl-(1-(4'-chlorophényl)-2,4,4-triméthyl-3-(1'',2'',4''-triazol-1''-yl)-pentyl-1)-éther.

4. Procédé de préparation d'un dérivé triazolylique selon la revendication 1, caractérisé par le fait que:

a) on fait réagir du 1,2,4-triazole avec des cétones insaturées en α, β de formule (II)

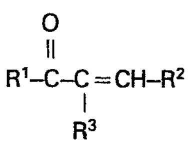

(II)

dans laquelle $R^1$, $R^2$ et $R^3$ ont la signification donnée dans la revendication 1 éventuellement en présence d'un solvant ou diluant et éventuellement en présence d'un catalyseur basique, et éventuellement

b) on réduit, éventuellement en présence d'un solvant ou diluant, des β-triazolylcétones de formule (III)

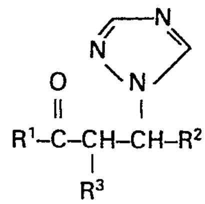

(III)

dans laquelle $R^1$, $R^2$ et $R^3$ ont les significations données dans la revendication 1, par voie catalytique avec de l'hydrogène ou avec des hydrures, pour les transformer en alcools de formule (IV)

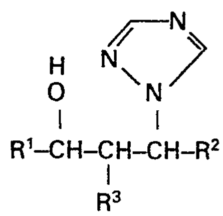

(IV)

c) on fait réagir des γ-triazolylalcools de formule (IV), dans laquelle $R^1$, $R^2$ et $R^3$ ont les significations données à la revendication 1.

α) avec un agent d'alkylation de Formule (V)

Hal–R$^4$ (V)

où Hal représente chlore, brome ou iode et $R^4$ a la signification donnée à la revendication 1, à l'exclusion d'hydrogène, ou

β) avec un halogénure d'acide de formule (VI)

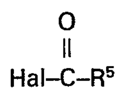

(VI)

où Hal représente chlore ou brome et $R^5$ le reste indiqué à la revendication 1, ou

γ) avec un isocyanate de formule (VII)

O=C=N–R$^5$ (VII)

où $R^5$ a la signification donnée à la revendication 1, les réactions α, β et γ étant effectuées éventuellement en présence d'un solvant ou diluant éventuellement en présence de bases inorganiques ou organiques, ainsi qu'éventuellement en présence d'un accélérateur de réaction, et on fait réagir les composés obtenus selon le procédé a) à c), éventuellement avec des acides ou des sels métalliques.

5. Moyen pour influer sur la croissance des plantes contenant un dérivé triazolylique selon la revendication 1, 2 ou 3.

6. Moyen pour influer sur la croissance des plantes contenant un support solide ou liquide et un dérivé triazolylique selon la revendication 1.

7. Procédé de préparation d'un moyen pour influer sur la croissance des plantes selon la revendication 6, caractérisé par le fait qu'on mélange un support solide ou liquide avec un dérivé triazolylique selon la revendication 1.

8. Procédé pour influer sur la croissance des plantes, caractérisé par le fait qu'on traite les plantes, leurs graines ou le sol avec un dérivé triazolylique selon la revendication 1.

**Revendications pour l'Etat Contractant: AT**

1. Moyen pour influer sur la croissance des plantes contenant un dérivé triazolylique de formule (I)

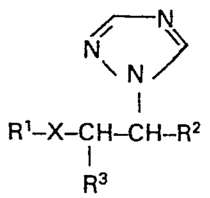

(I)

dans laquelle

$R^1$ et $R^2$ représentent, indépendamment l'un de l'autre, alkyle à 1 à 6 atomes de C, naphtyle, ou phényle, le rest phényle pouvant être substitué par du fluor, chlore, brome, alkyle ou alcoxy à 1 à 4 atomes de C et

$R^3$ représente alkyle à 1 à 4 atomes C et

X représente C=O ou H–C–$OR^4$, où $R^4$ représente un atome d'hydrogène, un reste alkyle à 1 à 4 atomes C, un reste allyle, un reste benzyle éventuellement substitué par du chlore ou du brome, un reste CO–$R^5$ ou un reste CO–$NHR^5$ dans lequel $R^5$ représente un reste alkyle à 1 à 4 atomes C ou un reste phényle éventuellement substitué par du chlore, brome ou alkyle à 1 à 4 atomes C,

ainsi que leurs sels et complexes métalliques.

2. Moyen pour influer sur la croissance des plantes selon la revendication 1, caractérisé par le fait que le dérivé triazolylique est 4-chlorophényl-(3'-(1'',2'',4''-triazol-1''-yl)-4',4'-diméthylpentyl-2'-)-cétone.

3. Moyen pour influer sur la croissance des plantes selon la revendication 1, caractérisé par le fait que le dérivé triazolylique est méthyl-(1-(4'-chlorophényl)-2,4,4-triméthyl -3-(1'',2'',4''-triazol-1''-yl)-pentyl-1)-éther.

4. Procédé de préparation d'un dérivé triazolylique tel que défini à la revendication 1, caractérisé par le fait que:

a) on fait réagir du 1,2,4-triazole avec des cétones insaturées en α, β de formule (II)

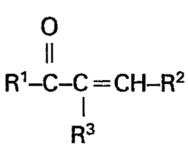

(II)

dans laquelle $R^1$, $R^2$ et $R^3$ ont la signification donnée dans la revendication 1, éventuellement en présence d'un solvant ou diluant et éventuellement en présence d'un catalyseur basique, et éventuellement

b) on réduit, éventuellement en présence d'un solvant ou diluant, des β-triazolylcétones de formule (III)

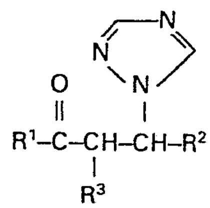

(III)

dans laquelle $R^1$, $R^2$ et $R^3$ ont les significations données dans la revendication 1, par voie catalytique avec de l'hydrogène ou avec des hydrures, pour les transformer en alcools de formule (IV)

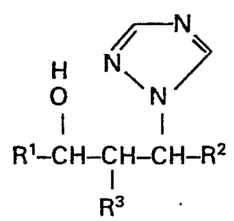

(IV)

et

c) on fait réagir des γ-triazolylalcools de formule (IV) dans laquelle $R^1$, $R^2$ et $R^3$ ont les significations données à la revendication 1,

α) avec un agent d'alkylation de formule (V)

$$Hal–R^4 \qquad (V)$$

où Hal représente chlore, brome ou iode et $R^4$ a la signification donné à la revendication 1, à l'exclusion d'hydrogène, ou

β) avec un halogénure d'acide de formule (VI)

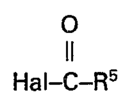

(VI)

où Hal représente chlore ou brome et $R^5$ le reste indiqué à la revendication 1, ou

γ) avec un isocyanate de formule (VII)

$$O=C=N–R^5 \qquad (VII)$$

où $R^5$ a la signification donnée à la revendication 1, les réactions α, β et γ étant effectuées éventuellement en présence d'un solvant ou diluant éventuellement en présence de bases inorganiques ou organiques, ainsi qu' éventuellement en présence d'un accélérateur de réaction, et on fait réagir les composés obtenus selon le procédé a) à c), éventuellement avec des acides ou des sels métalliques.

5. Moyen pour influer sur la croissance des

plantes contenant un support solide ou liquide et un dérivé triazolylique tel que défini à la revendication 1.

6. Procédé de préparation d'un moyen pour influer sur la croissance des plantes, caractérisé par le fait qu'on mélange un support solide ou liquide avec un dérivé triazolylique tel que défini à la revendication 1.

7. Procédé pour influer sur la croissance des plantes, caractérisé par le fait qu'on traite les plantes, leurs graines ou le sol avec un dérivé triazolylique défini selon la revendication.

**Claims for the Contracting States**
**BE, CH, DE, FR, GB, IT, LI, LU, NL and SE**

1. Triazolyl derivatives of the formula (I)

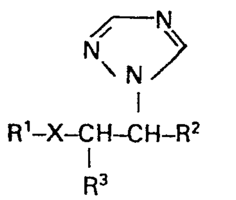

(I)

where $R^1$ and $R^2$ are identical or different and each denotes alkyl of from 1 to 6 carbon atoms, naphthyl, unsubstituted phenyl or phenyl substituted by fluoro, chloro, bromo, alkyl or alkoxy of from 1 to 4 carbon atoms, $R^3$ denotes alkyl of from 1 to 4 carbon atoms, and X denotes C=O or H–C–$OR^4$, $R^4$ denoting hydrogen, alkyl of from 1 to 4 carbon atoms, allyl, unsubstituted or chloro- or bromo-substituted benzyl, CO–$R^5$ or CO–$NHR^5$, $R^5$ denoting alkyl of from 1 to 4 carbon atoms, unsubstituted phenyl or phenyl substituted by chloro, bromo or alkyl of from 1 to 4 carbon atoms, and the salts and metal complexes thereof.

2. 4-Chlorophenyl-(3'-(1'',2'',4''-triazol-1''-yl)-4', 4'-dimethylpent-2'-yl)-ketone.

3.Methyl-(1-(4'-chlorophenyl)-2,4,4-trimethyl-3-(1'',2'',4''-triazol-1''-yl)-pent-1-yl)-ether.

4. Process for the production of a triazolyl derivate as claimed in claim 1, characerized in that
a) 1,2,4-triazole is reacted with α, β-unsaturated ketones of the formula (II)

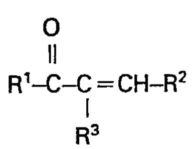

(II)

where $R^1$, $R^2$ and $R^3$ have the meanings given in claim 1, in the presence or absence of a solvent or diluent and in the presence or absence of a basic catalyst, and, if desired,
b) β-triazolyl ketones of the formula (III)

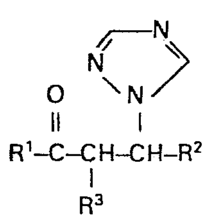

(III)

where $R^1$, $R^2$ and $R^3$ have the meanings given in claim 1 are catalytically reduced with hydrogen or hydrides to give alcohols of the formula (IV)

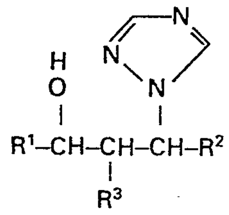

(IV)

in the presence or absence of a solvent or diluent, and
c) γ-triazolyl alcohols of the formula (IV), where $R^1$, $R^2$ and $R^3$ have the meanings given in claim 1, are reacted
α) with an alkylating agent of the formula (V)

Hal–$R^4$ (V)

where Hal denotes chlorine, bromine or iodine and $R^4$ has the meanings given in claim 1 with the exception of hydrogen, or
β) with an acid halide of the formula (VI)

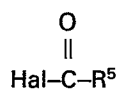

(VI)

where Hal denotes chlorine or bromine and $R^5$ has the meanings given in claim 1, or
γ) with an isocyanate of the formula (VII)

O=C=N–$R^5$ (VII)

where $R^5$ has the meanings given in claim 1, reactions α, β and γ taking place in the presence or absence of a solvent or diluent, in the presence or absence of inorganic or organic bases, and in the presence or absence of a reaction accelerator, and, if desired, the compounds obtained by processes a) to c) are reacted with acids or metal salts.

5. An agent for influencing plant growth containing a triazolyl derivative as claimed in claim 1, 2 or 3.

6. An agent for influencing plant growth containing a solid or liquid carrier and a triazolyl derivative as claimed in claim 1.

7. A process for the production of an agent for influencing plant growth as claimed in claim 6, characterized in that a solid or liquid carrier is mixed with a triazolyl derivative as claimed in claim 1.

8. A process for influencing plant growth, characterized in that the plants, their seed or the soil are treated with a triazolyl derivative as claimed in claim 1.

**Claims for the Contracting State: AT**

1. An agent for influencing plant growth containing a triazolyl derivative of the formula (I)

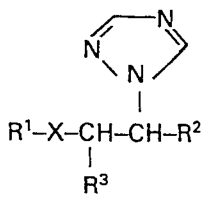

(I)

where $R^1$ and $R^2$ are identical or different and each denotes alkyl of from 1 to 6 carbon atoms, naphthyl, unsubstituted phenyl or phenyl substituted by fluoro, chloro, bromo, alkyl or alkoxy of from 1 to 4 carbon atoms, $R^3$ denotes alkyl of from 1 to 4 carbon atoms, and X denotes C=O or $H-C-OR^4$, $R^4$ denoting hydrogen, alkyl of from 1 to 4 carbon atoms, allyl, unsubstituted or chloro- or bromo-substituted benzyl, $CO-R^5$ or $CO-NHR^5$, $R^5$ denoting alkyl of from 1 to 4 carbon atoms, unsubstituted phenyl or phenyl substituted by chloro, bromo or alkyl of from 1 to 4 carbon atoms, and the salts and metal complexes thereof.

2. An agent for influencing plant growth as claimed in claim 1, characterized in that the triazolyl derivative is 4-chlorophenyl-(3′-(1″,2″,4″-triazol-1″-yl)-4′,4′-dimethylpent-2′-yl)-ketone.

3. An agent for influencing plant growth as claimed in claim 1, characterized in that the triazolyl derivative ist methyl-(1-(4′-chlorophenyl)-2,4,4-trimethyl- 3(1″,2″,4″-triazol-1″-yl)-pent-1-yl)-ether.

4. A process for the production of a triazolyl derivative as defined in claim 1, characterized in that

a) 1,2,4-triazole is reacted with α, β -unsaturated ketones of the formula (II)

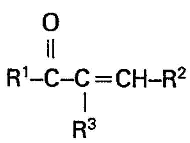

(II)

where $R^1$, $R^2$ and $R^3$ have the meanings given in claim 1, in the presence our absence of a solvent or diluent and in the presence or absence of a basic catalyst, and, if desired,

b) β-triazolyl ketones of the formula (III)

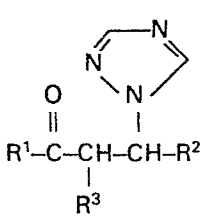

(III)

where $R^1$, $R^2$ and $R^3$ have the meanings given in claim 1 are catalytically reduced with hydrogen or hydrides to give alcohols of the formula (IV)

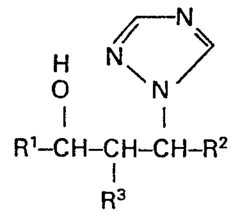

(IV)

in the presence or absence of a solvent or diluent, and

c) γ-triazolyl alcohols of the formula (IV), where $R^1$, $R^2$ and $R^3$ have the meanings given in claim 1, are reacted

α) with an alkylating agent of the formula (V)

$$Hal-R^4 \qquad (V)$$

where Hal denotes chlorine, bromine or iodine and $R^4$ has the meanings given in claim 1 with the exception of hydrogen, or

β) with an acid halide of the formula (VI)

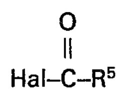

(VI)

where Hal denotes chlorine or bromine and $R^5$ has the meanings given in claim 1, or

γ) with an isocyanate of the formula (VII)

$$O=C=N-R^5 \qquad (VII)$$

where $R^5$ has the meanings given in claim 1, reactions α, β and γ taking place in the presence or absence of a solvent or diluent, in the presence or absence of inorganic or organic bases, and in the presence or absence of a reaction accelerator, and, if desired, the compounds obtained by processes a) to c) are reacted with acids or metal salts.

5. An agent for influencing plant growth containing a solid or liquid carrier and a triazolyl derivative as defined in claim 1.

6. A process for the production of an agent for influencing plant growth, characterized in that a solid or liquid carrier is mixed with a triazolyl derivative as defined in claim 1.

7. A process for influencing plant growth, characterized in that the plants, their seed or the soil are treated with a triazolyl derivative as defined in claim 1.